# EUROPEAN PATENT APPLICATION

(11) **EP 2 314 703 A1**
(43) Date of publication of application: **27.04.2011**
(21) Application number: 09013413.1
(22) Date of filing: 23.10.2009
(51) Int. Cl.: C12N 15/82

(54) **A plant promoter being tissue specific for endosperm and embryo of a plant seed**

(71) Applicant: Friedrich-Alexander-Universität Erlangen-Nürnberg, 91054 Erlangen (DE)
(72) Inventor: Sauer, Norbert, 91056 Erlangen (DE); Stadler, Ruth, 91056 Erlangen (DE)
(74) Representative: Engelhard, Markus

(57) **Abstract**

The present invention relates to a plant promoter being tissue specific for endosperm and embryo of a plant seed, in particular for the epidermis of an embryo of a plant seed, and to an expression system comprising such plant promoter. The present invention also relates to a vector comprising the plant promoter, and to a plant cell comprising the plant promoter or the aforementioned expression system or the aforementioned vector. Moreover, the present invention relates to a plant seed comprising such plant promoter or such expression system or such vector or such plant cell. The present invention also relates to a plant comprising a plant cell in accordance with the present invention. Also, the present invention relates to a method of selectively expressing a protein in endosperm and embryo of a plant seed, in particular in the epidermis of an embryo of a plant seed.

SEQUENCE LISTING

<110> Friedrich-Alexander-Universität Erlangen-Nürnberg

<120> A plant promoter being tissue specific for endosperm and embryo
of a plant seed

<130> FB22418

<160> 2

<170> PatentIn version 3.5

<210> 1
<211> 2150
<212> DNA
<213> Arabidopsis thaliana

<400> 1
ttgtttactg tttgcttcgg tagaacaaat atccaactag aaagtaattc cctctaccct 60

cagaaaagaa aattttgata ataatgtatg gcaattcttt tatatatagg gaacaagttg 120

aacaatttat gtagtttaga acggtttagg cgataaataa ccattaataa taccagttaa 180

attaattaat atgattaata attttaaaaa tacttattaa taaatattaa aacaattctc 240

atgtaagtac ataaaatata aacctagaag atattaaaat attaaatatt aaataagaaa 300

ccttaaatat tcttgcaaac aactagtcca atcaatatga catttttgta ttctataaac 360

acactgtatt ataatataaa ctatacagta tgtttcttag cacagtatga cacttaacaa 420

cttgatgatt tatactaggc aacaaccaat gtttttgaag atcatacaat atcatttata 480

agtatatcat tttttatatt acaaaatgaa agattgtttt ggtcaacaat atgcgggctt 540

gaaccctcag tggcgataca acattttaca tatctatata taagtttttt ccaccaattt 600

ttagaatatt tgcctaaaat ataagaatgt ttatactgct taattagtga gtaagaacag 660

gtagtccata tccattttat acaattcgga ctcagttgtc acgacttcgt taattggcaa 720

ctttggcaag tatgttgatt tcagaacaca tcgaatcaac aaaggacagt ctattgtgtt 780

aagttgcaaa cgatcaaaac aaggctggcg taacataaat gaaggccttt gggctaaata 840

aatataatag atacatcaaa atacaacttt caatttggac agacttgaag ccttctataa 900

tttggcctgg atcaatactc cttttaagac taaggtgtat cagcccagtc ttttgatttt 960

tgattatttt ttttgtaaac cgaggcaaga tttttgatag gcaataatcc tactttataa 1020

tacattcgga gtgtgtattc aattaaacat tttaaatgat ttgtgttaaa ataacaaatt 1080

cagcctcaca atttaatgtt aaggatacaa tcatatcatc ctttcattac ctcttatgca 1140

atttaccaca aatgattgaa acctcttatt caactagagg ttttgaaata ttgatatata 1200

tatttggatt ttgtaaataa ttaaggtttt taaattatgt catttaatta tttgaaaaat 1260

gagggtaaat aagattttgt acatgacttt ttttaaggag ttttaagttt ggcaattgtg 1320

atactacaaa tgatagtagt gtgacagtgt caccttggat tccattaatt ttctatgttc 1380

ccttttcatg aaattcatca aatagacaaa tacagtatta ttgtgcaaaa cccaaataaa 1440

tgaaaaggta aaatctaata aaccttggac cgtctatttc acccttcttt tagtcttctt 1500

actgacttta atttgaaata gaattggttt ttgtttttgg ccttcagtaa acattgtgtg 1560

taactaaagt cttcttttgt taacaagaaa gacccagaag atcacactag agaaaataag 1620

tcctttttat ggcacttctt tttgaattag atgggggaaa agttgaatta ctttacttta 1680

tttttattta ggtaaataaa ttacttaata tgacttcaaa aaatagacat taaataaaat 1740

ttgttaaact taattaattt gatatttgat taattcaaat tacttagaaa cattttaatt 1800

ctcatgtaaa gatactgacc aaaaaatatt aaaaagtata tactactaaa ataaggacgt 1860

ccgttaaatt ataaacctta aatattcttg taaacaacta agtttagtca actaaaaaaa 1920

acaaaacaaa gaggagttaa tgacctactg ccacgtgtcc tcacattgtc atgcactcac 1980

acaaatgaga tttctaactg cgtctcttat ctggaaaata atctcattcc ttcttcttcc 2040

tcgcctataa atacaactcc aatgtctcat ctcctacatc acaaaactaa atccccactt 2100

tcacaacaaa aaaactcttt tctttttgtc tgctcgtttt tcttttcata 2150

<210> 2
<211> 2030
<212> DNA
<213> Arabidopsis thaliana

<400> 2
aacaatttat gtagtttaga acggtttagg cgataaataa ccattaataa taccagttaa 60

attaattaat atgattaata attttaaaaa tacttattaa taaatattaa aacaattctc 120

atgtaagtac ataaaatata aacctagaag atattaaaat attaaatatt aaataagaaa 180

ccttaaatat tcttgcaaac aactagtcca atcaatatga catttttgta ttctataaac 240

acactgtatt ataatataaa ctatacagta tgtttcttag cacagtatga cacttaacaa 300

cttgatgatt tatactaggc aacaaccaat gtttttgaag atcatacaat atcatttata 360

agtatatcat tttttatatt acaaaatgaa agattgtttt ggtcaacaat atgcgggctt 420

gaaccctcag tggcgataca acattttaca tatctatata taagtttttt ccaccaattt 480

ttagaatatt tgcctaaaat ataagaatgt ttatactgct taattagtga gtaagaacag 540

gtagtccata tccattttat acaattcgga ctcagttgtc acgacttcgt taattggcaa 600

ctttggcaag tatgttgatt tcagaacaca tcgaatcaac aaaggacagt ctattgtgtt 660

aagttgcaaa cgatcaaaac aaggctggcg taacataaat gaaggccttt gggctaaata 720

aatataatag atacatcaaa atacaacttt caatttggac agacttgaag ccttctataa 780

tttggcctgg atcaatactc cttttaagac taaggtgtat cagcccagtc ttttgatttt 840

tgattatttt ttttgtaaac cgaggcaaga tttttgatag gcaataatcc tactttataa 900

tacattcgga gtgtgtattc aattaaacat tttaaatgat ttgtgttaaa ataacaaatt 960

cagcctcaca atttaatgtt aaggatacaa tcatatcatc ctttcattac ctcttatgca 1020

atttaccaca aatgattgaa acctcttatt caactagagg ttttgaaata ttgatatata 1080

tatttggatt ttgtaaataa ttaaggtttt taaattatgt catttaatta tttgaaaaat 1140

gagggtaaat aagattttgt acatgacttt ttttaaggag ttttaagttt ggcaattgtg 1200

atactacaaa tgatagtagt gtgacagtgt caccttggat tccattaatt ttctatgttc 1260

ccttttcatg aaattcatca aatagacaaa tacagtatta ttgtgcaaaa cccaaataaa 1320

tgaaaaggta aaatctaata aaccttggac cgtctatttc acccttcttt tagtcttctt 1380

actgacttta atttgaaata gaattggttt ttgtttttgg ccttcagtaa acattgtgtg 1440

taactaaagt cttcttttgt taacaagaaa gacccagaag atcacactag agaaaataag 1500

tcctttttat ggcacttctt tttgaattag atgggggaaa agttgaatta ctttacttta 1560

tttttattta ggtaaataaa ttacttaata tgacttcaaa aaatagacat taaataaaat 1620

ttgttaaact taattaattt gatatttgat taattcaaat tacttagaaa cattttaatt 1680

ctcatgtaaa gatactgacc aaaaaatatt aaaaagtata tactactaaa ataaggacgt 1740

ccgttaaatt ataaacctta aatattcttg taaacaacta agtttagtca actaaaaaaa 1800

acaaaacaaa gaggagttaa tgacctactg ccacgtgtcc tcacattgtc atgcactcac 1860

acaaatgaga tttctaactg cgtctcttat ctggaaaata atctcattcc ttcttcttcc 1920

tcgcctataa atacaactcc aatgtctcat ctcctacatc acaaaactaa atccccactt 1980

tcacaacaaa aaaactcttt tctttttgtc tgctcgtttt tcttttcata 2030

## Description

The present invention relates to a plant promoter being tissue specific for endosperm and embryo of a plant seed, in particular for the epidermis of an embryo of a plant seed, and to an expression system comprising such plant promoter. The present invention also relates to a vector comprising the plant promoter, and to a plant cell comprising the plant promoter or the aforementioned expression system or the aforementioned vector. Moreover, the present invention relates to a plant seed comprising such plant promoter or such expression system or such vector or such plant cell. The present invention also relates to a plant comprising a plant cell in accordance with the present invention. Also, the present invention relates to a method of selectively expressing a protein in endosperm and embryo of a plant seed, in particular in the epidermis of an embryo of a plant seed.

Many plants are of commercial interest because of their capability to produce specific compounds. These may be secondary metabolites, such as compounds having pharmacological activity, or they may e.g. be nutritional compounds, such as starch, sucrose, or fats.

As an example, in oleaginous plants, the compounds of commercial interest are oils, fats and lipids; these accumulate within the embryo and not in the endosperm of the seeds. Numerous attempts have been made to improve the yield of the oil production in such oleaginous plants. These concern specific selection of plants having a high oil yield. Other attempts have been made in optimizing the extraction efficiency with which the oil is extracted from the seeds. There remains a need in the art to increase the yields of production of desired compounds, such as oils, fats and lipids. One approach in this respect has been to increase the production of the desired compounds, e.g. the oil production by the expression of genes which optimize the "biosynthetic fitness" of the plants, e. g. by increasing the import of nutrients into the seeds which are necessary for an optimum development thereof. In many instances, however, this could not be achieved, because the import of such nutrients was unspecific and not targeted.

Accordingly, it was an object of the present invention to provide for means that allow a specific and targeted expression of genes in the developing embryo and the endosperm of a plant seed, in particular in the epidermis of the embryo.

These objects are solved by a method of selectively expressing a protein in endosperm and embryo of a plant seed, in particular the epidermis of an embryo of a plant seed, said method comprising:
a) introducing into a plant cell a plant promoter being tissue specific for endosperm and embryo of a plant seed, in particular the epidermis of said embryo, together with a nucleic acid coding for a protein to be specifically expressed in the endosperm and embryo of a plant seed, and
b) generating a whole plant from said plant cell, and
c) obtaining seed from said whole plant,
wherein said nucleic acid coding for a protein to be specifically expressed in the endosperm and embryo of said plant seed is arranged downstream of said plant promoter and is connected to said plant promoter in a sense direction, such that said plant promoter directs expression of said protein coded by said nucleic acid, and
wherein said plant promoter has a nucleic acid sequence consisting of 100-200 contiguous nucleotides, or 200-300 contiguous nucleotides, or 300-400 contiguous nucleotides, or 400-500 contiguous nucleotides, or 500-600 contiguous nucleotides, or 600-700 contiguous nucleotides, or 700-800 contiguous nucleotides, or 800-900 contiguous nucleotides, or 900-1000 contiguous nucleotides, or 1000-1100 contiguous nucleotides, or 1100-1200 contiguous nucleotides, or 1200-1300 contiguous nucleotides, or 1300-1400 contiguous nucleotides, or 1400-1500 contiguous nucleotides, or 1500-1600 contiguous nucleotides, or 1600-1700 contiguous nucleotides, or 1700-1800 contiguous nucleotides, or 1800-1900 contiguous nucleotides, or 1900-2000 contiguous nucleotides, or 2000-2100 contiguous nucleotides, or 2030-2149 contiguous nucleotides, or 2030-2150 contiguous nucleotides taken from SEQ ID NO: 1, or from a nucleic acid sequence which is at least 95 %, preferably at least 99 % identical to SEQ ID NO: 1.

In one embodiment, said plant promoter has a nucleic acid sequence represented by SEQ ID NO: 2, or a sequence which is at least 95 %, preferably at least 99 % identical to SEQ ID NO: 2.

In one embodiment, said plant promoter has a nucleic acid sequence represented by SEQ ID NO: 1, or a sequence which is at least 95 %, preferably at least 99 % identical to SEQ ID NO: 1.

In one embodiment, said plant cell is derived from a plant species selected from plants, the mature seeds of which do not contain an endosperm.

In one embodiment, said plant cell is derived from a plant species selected from oleaginous plants, such as rapeseed, leguminous plants, such as peas and beans, Asteraceae plants, such as sunflower, Cucurbitaceae plants, such as pumpkin, and Juglandaceae plants, such as walnut.

This object is also solved by a plant promoter being tissue specific for endosperm and embryo of a plant seed, in particular the epidermis of an embryo of a plant seed, said plant promoter having a nucleic acid sequence comprising at least 100, 200, 300, 400, 500, 600, 700, 800, 900, 1000, 1100, 1200, 1300, 1400, 1500, or 1505 contiguous nucleotides of SEQ ID NO: 1, preferably at least 1700 contiguous nucleotides, more preferably at least 1900 contiguous nucleotides, even more preferably at least 2000 contiguous nucleotides, and most preferably at least 2030 contiguous nucleotides of SEQ ID NO: 1, or of a sequence which is at least 95 %, preferably at least 99 % identical to SEQ ID NO: 1. Sequences which are "at least 95 %, preferably at least 99 % identical" to SEQ ID NO: 1 are those sequences which account for the intraspecies sequences variation which may occur. Such sequences are explicitly encompassed herein. A sequence W is said to be "X % identical to SEQ ID NO: 1 ", if X % residues of SEQ ID NO: 1 can be found in sequence W in corresponding positions.

In one embodiment, said plant promoter consists of
100-200 contiguous nucleotides, or of
200-300 contiguous nucleotides, or of
300-400 contiguous nucleotides, or of
400-500 contiguous nucleotides, or of
500-600 contiguous nucleotides, or of
600-700 contiguous nucleotides, or of
700-800 contiguous nucleotides, or of,
800-900 contiguous nucleotides, or of
900-1000 contiguous nucleotides, or of
1000-1100 contiguous nucleotides, or of
1100-1200 contiguous nucleotides, or of
1200-1300 contiguous nucleotides, or of
1300-1400 contiguous nucleotides, or of
1400-1500 contiguous nucleotides, or of
1505-1600 contiguous nucleotides, or of
1600-1700 contiguous nucleotides, or of
1700-1800 contiguous nucleotides, or of
1800-1900 contiguous nucleotides, or of
1900-2000 contiguous nucleotides, or of
2000-2100 contiguous nucleotides, or of
2100-2149 contiguous nucleotides, or of
2100-2150 contiguous nucleotides,

taken from SEQ ID NO: 1 or from a sequence which is at least 95 %, preferably at least 99 % identical to SEQ ID NO: 1.

In one embodiment the plant promoter has a nucleic acid sequence represented by SEQ ID NO: 2.

In one embodiment the plant promoter has a nucleic acid sequence represented by SEQ ID NO: 1.

The objects of the present invention are also solved by an expression system comprising a plant promoter according to the present invention and a nucleic acid coding for a protein to be specifically expressed in the endosperm or embryo of a plant seed, said nucleic acid coding for a protein being arranged downstream of said plant promoter and being connected to said plant promoter in a sense direction, such that said plant promoter directs expression of said protein coded by said nucleic acid.

The objects of the present invention are also solved by a vector comprising the plant promoter according to the present invention or the expression system according to the present invention.

The objects of the present invention are also solved by a plant cell comprising the plant promoter according to the present invention or the expression system according to the present invention or the vector according to the present invention.

In one embodiment the plant cell according to the present invention is derived from a plant species selected from plants, the mature seeds of which do not contain an endosperm. A person skilled in the art knows or is able to determine whether a mature plant seed contains an endosperm. In one embodiment, the plant cell according to the present invention is derived from a plant species selected from oleaginous plants, such as rapeseed, soybean, cotton, peanut, sesame, leguminous plants, such as pea and bean, Asteraceae plants, such as sunflower, Cucurbitaceae plants, such as pumpkin, and Juglandaceae plants, such as walnut.

The objects of the present invention are also solved by a plant seed comprising the plant promoter according to the present invention or the expression system according to the present invention or the vector according to the present invention or at least one plant cell according to the present invention. Preferably, the plant seed is derived from a plant species selected from plants as defined above.

The objects of the present invention are also solved by a plant comprising a plant cell according to the present invention.

In one embodiment the plant is derived from a plant species selected from plants as defined above.

In one embodiment, the plant according to the present invention comprises the plant promoter according to the present invention, wherein said plant promoter has been deliberately introduced into said plant. Likewise, in one embodiment, the plant cell according to the present invention comprises the plant promoter according to the present invention, wherein said plant promoter has been deliberately introduced into said plant cell. Likewise, in one embodiment, the plant seed according to the present invention comprises the plant promoter according to the present invention, wherein said plant promoter has been deliberately introduced into said plant seed.

Methods of artificially or deliberately introducing nucleic acids into plants/plant cells are known to a person skilled in the art and include, amongst others, Agrobacterium mediated transformation, biolistic particle bombardment, and viral transformation.

The objects of the present invention are also solved by a method of selectively expressing a protein in endosperm and embryo of a plant seed, in particular in the epidermis of an embryo of a plant seed, said method comprising:
a) introducing an expression system according to the present invention or a vector according to the present invention into a plant cell, and
b) generating a whole plant from said plant cell, and
c) obtaining seed from said whole plant.

The plant cell and plant, in this aspect of the present invention, are as defined further above.

In accordance with embodiments of the present invention, a plant promoter can be used to direct a tissue specific expression of proteins of interest in the endosperm and embryo of a plant seed, in particular in the epidermis of the embryo of a plant seed. In one embodiment, the plant promoter has a nucleic acid sequence which is represented by SEQ ID NO: 2. In one embodiment, the plant promoter consists of the nucleic acid sequence represented by SEQ ID NO:2. In one embodiment, the plant promoter has a nucleic acid sequence represented by SEQ ID NO: 1. In yet a further embodiment, the plant promoter according to the present invention consists of a nucleic acid sequence represented by SEQ ID NO: 1.

Proteins of interest that may be specifically expressed using the present invention are selected from carbohydrate transporters, such as sucrose transporters, and monosaccharide transporters, eventually also together with extracellular invertases. Other proteins that may be specifically expressed using the present invention are selected from proteins involved in the biosynthesis of oils, lipids and fats, such as acyltransferases, fatty acid elongases, acyl-CoA-synthetasis.

The plant promoter, in accordance with embodiments of the present invention is connected to the nucleic acid coding for the protein to be expressed in such a manner that the promoter directs expression of said protein. A person skilled in the art knows how to produce such functional constructs. Hence, the nucleic acid coding for the protein to be expressed is connected to the plant promoter in a sense direction downstream of said plant promoter.

The plant promoter in accordance with embodiments of the present invention is particularly useful in oleaginous plants, but also in plants of other species which may be used for commercial purposes. Accordingly, the plant cell, plant seed and whole plant is preferably derived from a plant species selected from Arabidopsis thaliana, Brassica napus, beans, peas, pumpkin, sunflower, walnut, cotton, soybean, peanut, sesame. The term "derived from a plant species", when used in connection with a plant or plant cell herein, is meant to refer to such plant or plant cell belonging to such plant species. The term is also meant to include such plants and plant cells the genetic make-up of which is that of the indicated species, although the plant or plant cell may also contain nucleic acids which are foreign to such species. Hence, the term "derived from a plant species" is meant to also include transgenic plants and plant cells.

The present invention also relates to the use of a plant promoter as defined above for the tissue specific expression of proteins of interest in the endosperm and embryo of a plant seed, in particular in the epidermis of the embryo of a plant seed, the plant promoter being as defined further above. Preferably such use comprises the incorporation of the plant promoter, together with the nucleic acid coding for a protein of interest into a plant cell, generating a whole plant from such plant cell and obtaining the seeds from such whole plant. Preferably, said use in accordance with embodiments of the present invention is for a specific expression of proteins of interest in the epidermis of the embryo and the endosperm of a plant seed.

The method and use in accordance with the present invention are particularly useful in oleaginous plants, such as Brassica napus (rapeseed), but may also be used with other plants of commercial interest, such as soybean, cotton, peanut, sesame, pea, bean, pumpkin, sunflower, walnut, olive.

The present inventors have surprisingly found that a stretch of the promoter of the AtSUC5 protein from Arabidopsis thaliana directs the expression of a protein of interest to the endosperm and the epidermis of the developing embryo. Previously, the Arabidopsis thaliana AtSUC5 protein had been characterized as a transport protein of the plant plasma membrane catalyzing the transport of biotin and sucrose. Baud et al., 2005, The Plant Journal, 43, 824-836 found that the sucrose transporter gene AtSUC5 was only expressed in the endosperm of Arabidopsis thaliana seeds, i. e. the nutritional tissue surrounding the developing embryo. In contrast thereto, the present inventors could show that the promoter in accordance with the present invention directs the expression of a gene of interest to the endosperm and, what had not been previously described, to the epidermis (i. e. the outermost cell layer of the embryo). Such expression in the embryo epidermis appears to start at a time, when the oil production within the embryo is initiated and is restricted to the physiological underside of the embryo cotyledons, that is to the cell layer that has contacts to the surrounding endosperm which is therefore responsible for the transfer of compounds from the endosperm to the embryo. The plant promoter in accordance with the present invention there provides for an ideal temporal as well as special resolution of expression.

Moreover, reference is made to the enclosed sequence listing, wherein,
SEQ ID NO: 1 represents the 2150 bp sequence upstream of At 1g 71890 and downstream of At 1g 71880;
SEQ ID NO: 2 represents one example of a stretch of 2030 bp taken from SEQ ID NO: 1.

The present inventors envisage that any stretch as small as 100 contiguous nucleotides, preferably 100-200, or 200-300, or 300-400, or 400-500, or 500-600, or 600-700, or 700-800, or 800-900, or 900-1000, or 1000-1100, or 1100-1200, or 1200-1300, or 1300-1400, or 1400-1500, or 1500-1600, or 1600-1700, or 1700-1800, or 1800-1900, or 1900-2000, or 2000-2100, or 2100-2149, or 2100-2150 contiguous nucleotides taken from SEQ ID NO: 1, or from a sequence that is at least 95 %, preferably at least 99 % identical to SEQ ID NO: 1, will allow for the required tissue specific expression. In one embodiment, a stretch of at least 1505 contiguous nucleotides, preferably at least 1700 contiguous nucleotides, more preferably at least 1900 contiguous nucleotides, even more preferably at least 2000 contiguous nucleotides, and most preferably at least 2030 contiguous nucleotides, taken from SEQ ID NO: 1, or from a sequence that is at least 95 %, preferably at least 99 % identical to SEQ ID NO: 1, will allow for the required tissue specific expression, in particular for an expression in the endosperm and embryo of a plant seed, more particularly in the epidermis of the embryo.

Moreover, reference is made to the enclosed figure, wherein

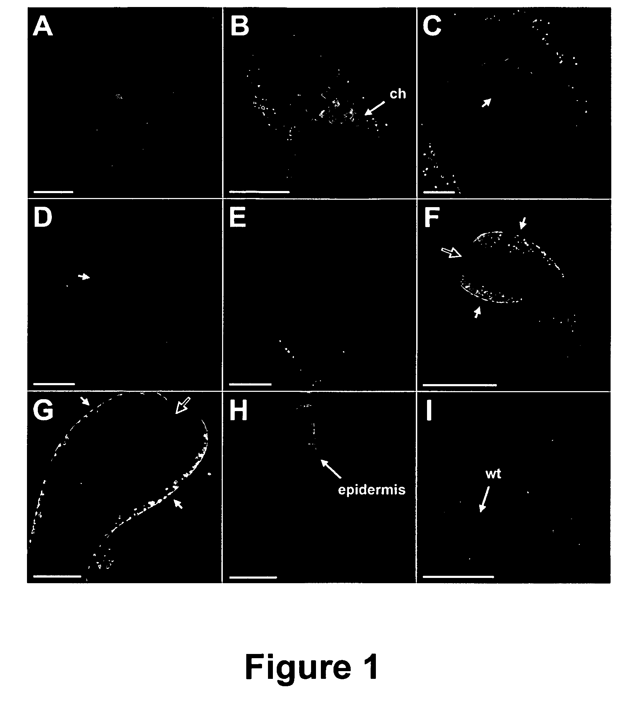

Figure 1 shows analyses of pAtSUC5/sGFP plants and pAtSUC5/tmGFP9 plants.

More specifically, figure 1 shows confocal images of developing seeds [(**A**) to (**E**)] and isolated embryos [(**F**) to (**I**)] from *pAtSUC5lsGFP* plants [(**A**), (**D**) and (**E**)] or *pAtSUC5*/*tmGFP9* [(**B**), (**C**), and (**F**) to (**I**)] plants are presented.
(**A**) Young seed with syncytial endosperm and no detectable embryo showing GFP fluorescence in the endosperm nuclei (maximal projection).
(**B**) Young seed with syncytial endosperm showing strong GFP fluorescence in the chalazal region (maximal projection; ch).
(**C**) Developing seed showing GFP fluorescence (optical section) in the ER of the endosperm but not in the globular embryo (arrow).
(**D**) Slightly older seed with GFP fluorescence in the endosperm nuclei (maximal projection) but not in the heart-stage embryo (arrow).
(**E**) Developing seed with GFP fluorescence in the early-torpedo-stage embryo and in the endosperm.
(**F**) Isolated midtorpedo-stage embryo (maximal projection) showing highest GFP fluorescence at the physiological underside of the forming cotyledons (white arrows) and, no GFP fluorescence on the upper side (black arrow), and little fluorescence in the radicle.
(**G**) Higher magnification of an embryo (optical section) at the same developmental stage as shown in (**F**).
(**H**) Higher magnification (optical section) of e section through the underside of a forming cotyledon [same embryo as in (**G**)] showing GFP fluorescence only in the epidermis.
(**I**) Comparison of the fluorescence in an isolated wt embryo [slightly older than the embryo shown in (**E**)] and *pAtSUC5*/*tmGFP9* embryo [similar stage as in (**F**) and (**G**)].
Red color results from autofluorescence of chlorophyll. Scale bars are 10 µm in (**H**), 25 µm in (**C**), 50 µm in (**A**), 100 µm in (**B**), (**D**), (**E**) and (**G**), and 200 µm in (**F**) and (**I**).
Moreover, reference is made to the example which is given to illustrate, not to limit the present invention.

### Example

### Analyses of pAtSUC5/sGFP plants and pAtSUC5/tmGFP9 plants

The present inventors generated *AtSUC5* promoter (pAtSUC5)/reporter lines that expressed the open reading frames (ORFs) of the soluble and freely mobile green fluorescent protein (sGFP) or of a membrane-bound version of GFP (tmGFP9) that is linked to several transmembrane helices and, therefore, membrane-localized and immobile (Stadler et al., 2005 Physiol. 139, 704-712) under the control of a 2030-bp *AtSUC5* promoter. The *pAtSUC5* fragment used in previous analyses by Baud et al. (2005, Plant J., 43, 824-836) was 1.5 kb long. After BASTA-selection of T1 seedlings, the present inventors obtained numerous transformed T1 plants for both constructs. Analyses of these plants by confocal microscopy confirmed the expression *of AtSUC5* in the endosperm with both constructs (Fig. 2A and 2B), and also the accumulation of GFP at the chalazal end of the endosperm (Fig. 2B) and the absence of GFP from globular-stage embryos (Fig. 2C) that were already published (Baud et al., 2005). Moreover, they observed no expression of *GFP* in heart-stage embryos (Fig. 2D). The present inventors did, however, find *pAtSUC5* activity with both constructs during later stages of embryo development (Fig. 2E to 2I). This embryo-specific expression has not been observed in earlier analyses performed with standard fluorescence microscopy and a shorter promoter fragment (Baud et al., 2005).

Optical sections through *pAtSUC5*/*tmGFP9* embryos confined the *pAtSUC5* activity to the embryo epidermis (Fig. 2G and 2H). In previous analyses (Stadler et al., 2005) it had been shown that sGFP synthesized in the embryo epidermis from Arabidopsis *GLABRA2* promoter *(pAtGL2)*/*sGFP* constructs can move symplastically into all other cells of the developing embryo due to the presence of large plasmodesmata. This explains the homogenous fluorescence seen in the early-torpedo-stage embryo expressing *sGFP* from p*AtSUC5* (Fig. 2E). Wild type embryos did not show fluorescence at any developmental stage (Fig. 2I).

The features of the present invention disclosed in the specification, the claims, and/or in the accompanying drawings may, both separately and in any combination thereof, be material for realizing the invention in various forms thereof.

## Claims

1. A method of selectively expressing a protein in endosperm and embryo of a plant seed, in particular the epidermis of an embryo of a plant seed, said method comprising:
a) introducing into a plant cell a plant promoter being tissue specific for endosperm and embryo of a plant seed, in particular the epidermis of said embryo, together with a nucleic acid coding for a protein to be specifically expressed in the endosperm and embryo of a plant seed, and
b) generating a whole plant from said plant cell, and
c) obtaining seed from said whole plant,
wherein said nucleic acid coding for a protein to be specifically expressed in the endosperm and embryo of said plant seed is arranged downstream of said plant promoter and is connected to said plant promoter in a sense direction, such that said plant promoter directs expression of said protein coded by said nucleic acid, and
wherein said plant promoter has a nucleic acid sequence consisting of 100-200 contiguous nucleotides, or 200-300 contiguous nucleotides, or 300-400 contiguous nucleotides, or 400-500 contiguous nucleotides, or 500-600 contiguous nucleotides, or 600-700 contiguous nucleotides, or 700-800 contiguous nucleotides, or 800-900 contiguous nucleotides, or 900-1000 contiguous nucleotides, or 1000-1100 contiguous nucleotides, or 1100-1200 contiguous nucleotides, or 1200-1300 contiguous nucleotides, or 1300-1400 contiguous nucleotides, or 1400-1500 contiguous nucleotides, or 1500-1600 contiguous nucleotides, or 1600-1700 contiguous nucleotides, or 1700-1800 contiguous nucleotides, or 1800-1900 contiguous nucleotides, or 1900-2000 contiguous nucleotides, or 2000-2100 contiguous nucleotides, or 2030-2149 contiguous nucleotides, or 2030-2150 contiguous nucleotides taken from SEQ ID NO: 1, or from a nucleic acid sequence which is at least 95 %, preferably at least 99 % identical to SEQ ID NO: 1.

2. The method according to claim 1, wherein said plant promoter has a nucleic acid sequence represented by SEQ ID NO: 2, or a sequence which is at least 95 %, preferably at least 99 % identical to SEQ ID NO: 2.

3. The method according to claim 1, wherein said plant promoter has a nucleic acid sequence represented by SEQ ID NO: 1, or a sequence which is at least 95 %, preferably at least 99 % identical to SEQ ID NO: 1.

4. The method according to any of the foregoing claims, wherein said plant cell is derived from a plant species selected from plants, the mature seeds of which do not contain an endosperm.

5. The method according to any of the foregoing claims, wherein said plant cell is derived from a plant species selected from oleaginous plants, such as rapeseed, leguminous plants, such as peas and beans, Asteraceae plants, such as sunflower, Cucurbitaceae plants, such as pumpkin, and Juglandaceae plants, such as walnut.

6. A plant promoter being tissue specific for endosperm and embryo of a plant seed, said plant promoter having a nucleic acid sequence consisting of 100-200 contiguous nucleotides, or 200-300 contiguous nucleotides, or 300-400 contiguous nucleotides, or 400-500 contiguous nucleotides, or 500-600 contiguous nucleotides, or 600-700 contiguous nucleotides, or 700-800 contiguous nucleotides, or 800-900 contiguous nucleotides, or 900-1000 contiguous nucleotides, or 1000-1100 contiguous nucleotides, or 1100-1200 contiguous nucleotides, or 1200-1300 contiguous nucleotides, or 1300-1400 contiguous nucleotides, or 1400-1500 contiguous nucleotides, or 1505-1600 contiguous nucleotides, or 1600-1700 contiguous nucleotides, or 1700-1800 contiguous nucleotides, or 1800-1900 contiguous nucleotides, or 1900-2000 contiguous nucleotides, or 2000-2100 contiguous nucleotides or 2030-2149 contiguous nucleotides, or 2030-2150 contiguous nucleotides taken from SEQ ID NO: 1, or from a nucleic acid sequence which is at least 95 %, preferably at least 99 % identical to SEQ ID NO: 1.

7. The plant promoter according to claim 6, having a nucleic acid sequence represented by SEQ ID NO: 2, or a sequence which is at least 95 %, preferably at least 99 % identical to SEQ ID NO: 2.

8. The plant promoter according to claim 6, having a nucleic acid sequence represented by SEQ ID NO: 1, or a sequence which is at least 95 %, preferably at least 99 % identical to SEQ ID NO: 1.

9. An expression system comprising a plant promoter according to any of claims 6-8 and a nucleic acid coding for a protein to be specifically expressed in the endosperm and embryo of a plant seed, said nucleic acid coding for a protein being arranged downstream of said plant promoter and being connected to said plant promoter in a sense direction, such that said plant promoter directs expression of said protein coded by said nucleic acid.

10. A vector comprising the plant promoter according to any of claims 6-8 or the expression system according to claim 9.

11. A plant cell comprising the plant promoter according to any of claims 6-8 or the expression system according to claim 9 or the vector according to claim 10.

12. A plant seed comprising the plant promoter according to any of claims 6-8 or the expression system according to claim 9 or the vector according to claim 10 or at least one plant cell according to claim 11.

13. A plant comprising a plant cell according to claim 11.

14. The plant cell according to claim 11 or the plant seed according to claim 12 or the plant according to claim 13, being derived from a plant species selected from plants, the mature seeds of which do not contain an endosperm.

15. The plant cell according to claim 11 or the plant seed according to claim 12 or the plant according to claim 13, being derived from a plant species selected from oleaginous plants, such as rapeseed, leguminous plants, such as peas and beans, Asteraceae plants, such as sunflower, Cucurbitaceae plants, such as pumpkin, and Juglandaceae plants, such as walnut.
